# EUROPEAN PATENT SPECIFICATION

(11) **EP 3 845 235 B1**
(45) Date of publication and mention of the grant of the patent: **01.04.2026**
(21) Application number: 19823524.4
(22) Date of filing: 10.06.2019
(51) Int. Cl.: A23L 33/105, A61K 36/605, A61K 36/736, A61P 25/00, A61P 9/10

(54) **COMPOSITION FOR NEUROPROTECTION, CONTAINING PLANT EXTRACT OR FRACTION AS ACTIVE INGREDIENT**
MITTEL ZUR NEUROPROTEKTION MIT PFLANZENEXTRAKT ODER -FRAKTION ALS WIRKSTOFF
COMPOSITION DE NEUROPROTECTION, CONTENANT UN EXTRAIT OU UNE FRACTION VÉGÉTAL(E) COMME INGRÉDIENT ACTIF

(30) Priority: 18.06.2018 KR 20180069854; 09.07.2018 KR 20180079491
(43) Date of publication of application: 07.07.2021
(73) Proprietor: Sookmyung Women's University Industry-Academic Cooperation Foundation, Seoul 04310 (KR)
(72) Inventor: SONG, Yun Seon, Seoul 06518 (KR); CHANG, Min Sun, Seoul 04360 (KR); KIM, So Dam, Seoul 08505 (KR); LIM, Da Sol, Cheonan-si Chungcheongnam-do 31179 (KR); YUN, You Jung, Seoul 07028 (KR); KANG, Su Min, Bucheon-si Gyeonggi-do 14549 (KR); CHOI, Sang Ho, Daejeon 34141 (KR); EUM, Sang Mi, Daejeon 34141 (KR); KIM, Soo Yong, Daejeon 34141 (KR); PAIK, Jin Hyub, Daejeon 34141 (KR); KIM, Seo Hea, Incheon 21977 (KR); LEE, Na Kyung, Seoul 04310 (KR); KIM, Youn In, Daejeon 34141 (KR); LEE, Sang Woo, Daejeon 34141 (KR); JIN, Hang, Kunming City, Yunnan 650000 (CN); LI, Wan Yi, Kunming City, Yunnan 650000 (CN)
(74) Representative: Algemeen Octrooi- en Merkenbureau B.V.
(86) International application number: PCT/KR2019/006942
(87) International publication number: WO 2019/245207

(56) References cited:
- KR-A- 20170 043 232
- KR-A- 20170 043 232
- US-A1- 2008 292 607
- US-A1- 2008 292 607
- DA SOL LIM: "Neuroprotective effect of Maclura Pubescens as a phytoestrogen via neuroglobin(Ngb) after tMCAO in mice", DISSERTATION- DEPARTMENT OF PHARMACY PHARMACO THERAPY MAJOR, 1 December 2017 (2017-12-01), pages 1 - 67, XP055664991
- SO DAM KIM: "Neuroprotective effect of Prunus cerasoides via estrogen receptor (ER) neuroglobin upregulation", DISSERTATION- DEPARTMENT OF PHARMACY. PHARMACO THERAPY MAJOR, 1 December 2016 (2016-12-01), pages 1 - 89, XP055665003
- KEITH R. MARTIN ET AL: "Tart Cherry Juice Induces Differential Dose-Dependent Effects on Apoptosis, But Not Cellular Proliferation, in MCF-7 Human Breast Cancer Cells", JOURNAL OF MEDICINAL FOOD, vol. 15, no. 11, 1 November 2012 (2012-11-01), US, pages 945 - 954, XP055377690, ISSN: 1096-620X, DOI: 10.1089/jmf.2011.0336
- HONG SUNGEUN ET AL: "The isoflavones and extracts fromMaclura tricuspidatafruit protect against neuronal cell death in ischemic injury via induction of Nox4-targeting miRNA-25, miRNA-92a, and miRNA-146a", JOURNAL OF FUNCTIONAL FOODS, ELSEVIER BV, NL, vol. 40, 22 December 2017 (2017-12-22), pages 785 - 797, XP085357991, ISSN: 1756-4646, DOI: 10.1016/J.JFF.2017.12.011
- BELWAL TARUN ET AL: "A critical analysis of extraction techniques used for botanicals: Trends, priorities, industrial uses and optimization strategies", TRAC TRENDS IN ANALYTICAL CHEMISTRY, vol. 100, 1 March 2018 (2018-03-01), AMSTERDAM, NL, pages 82 - 102, XP055784685, ISSN: 0165-9936, DOI: 10.1016/j.trac.2017.12.018
- LIM DA SOL: "Neuroprotective effect of Maclura Pubescens as a phytoestrogen via neuroglobin(Ngb) after tMCAO in mice", DISSERTATION- DEPARTMENT OF PHARMACY PHARMACO THERAPY MAJOR, December 2017 (2017-12-01) - February 2018 (2018-02-01), pages 1 - 67, XP055664991
- KIM SO DAM: "Neuroprotective effect of Prunus cerasoides via estrogen receptor (ER) neuroglobin upregulation", DISSERTATION- DEPARTMENT OF PHARMACY. PHARMACO THERAPY MAJOR, December 2016 (2016-12-01) - February 2017 (2017-02-01), Graduate School of Sookmyung Women's University, pages 1 - 89, XP055665003
- MARTIN, K. R.: "Tart cherry juice induces differential dose-dependent effects on apoptosis, but not cellular proliferation, in MCF-7 human breast cancer cells", JOURNAL OF MEDICINAL FOOD, vol. 15, no. 11, 2012, pages 945 - 954, XP055377690, DOI: 10.1089/jmf.2011.0336

## Description

### [Technical Field]

The present disclosure relates to a composition for neuroprotection, containing a Prunus cerasoides extract or fraction, as active ingredients.

### [Background Art]

Stroke refers to a case in which a partial or total rapid impairment in brain function occurs and this condition persists for a considerable period of time or longer, and other causes other than cerebrovascular disease cannot be found. In oriental medicine, stroke is also referred to as 'jungpung' or 'pung', but jungpung is not a consistent concept with stroke because it includes diseases that are not included in stroke. Stroke is a term that includes both cerebral infarction (ischemic stroke) and cerebral hemorrhage (hemorrhagic stroke) caused by bleeding of blood into brain tissue due to rupture of cerebrovascular vessels.

The brain tissue is usually supplied with a large amount of blood flow, but the cerebrovascular vessels are blocked due to various causes, and when the amount of blood supplied to the brain decreases, the brain tissue is not function properly. If the decrease in cerebral blood flow continues for a certain period of time, the brain tissue is subject to necrosis, and due to the necrosis of the brain tissue, reaching a state that cannot be recovered is called cerebral infarction. Cerebral infarction includes cerebral thrombosis and cerebral embolism. Thrombosis refers to the blockage of blood vessels due to the hardening of blood in a local area of the blood vessel. If a blood clot occurs, the blood flow is cut off in the area of the blood vessel, and if the complementary supply of blood from the other area is not enough, the brain tissue in the area is killed. Embolism refers to a blockage of a peripheral blood vessel by a blood clot occurring in the heart being carried to the peripheral by the blood flow. If the blood vessel is blocked by embolus, the brain tissue in the area is killed. In general, it is known that the embolus is easier to be dissolved than blood clots, and the blood flow is often reflowed in many cases.

Degenerative diseases of the brain include Alzheimer's disease, Wilson disease, and Parkinson disease, etc., which show commonly diffuse brain atrophy. Degenerative diseases that invade the cerebral cortex include Alzheimer's disease and Pick's disease, etc., and the main symptom is dementia.

Dementia by itself does not mean a disease, but is a generic term that refers to a condition in which various cognitive dysfunctions, including memory, are caused by brain damage from several causes, and thus the previous level of daily life cannot be maintained. Dementia refers to a complex symptom in which the normal mature brain is damaged or destroyed by an external cause such as acquired trauma or disease, etc., and the cognitive and advanced mental functions such as intelligence, learning, language, etc., are generally deteriorated. The most common cause of dementia is Alzheimer's dementia (AD), which accounts for about 50 to 60%, followed by vascular dementia (VaD), which accounts for 20 to 30%, and the dementia due to other causes, which accounts for 10 to 30%. Vascular dementia is a cerebrovascular disease such as cerebral infarction or cerebral hemorrhage, etc., which is caused by clogging or narrowing of cerebrovascular vessels.

Drugs applied to degenerative diseases of the brain include acetylcholinesterase-inhibiting drugs such as tacrine, vitamin E that inhibits the destruction of brain cells by active oxygen, and antioxidants such as selegiline. However, drugs developed so far are known to have insufficient efficacy and have a significant number of side effects.

In addition, the general population complaining of decreased memory and concentration is increasing, even if it is not the above-mentioned degenerative diseases. Therefore, there is a need for drugs that can improve brain function and memory, with no or minimal side effects.

Neuroglobin (Ngb) is a subtype of globin found in neurons, has a function of detecting, binding and transporting O₂ in neurons, and is involved in scavenge of reactive oxygen species (ROS). It has been reported that Ngb promotes nerve regeneration, and it has been confirmed that Ngb is expressed at a very early stage of nerve differentiation, and may promote nerve regeneration by preserving mitochondrial function and ATP production. In addition, it is known that Ngb plays an important role in cellular respiration through oxygen transport to brain tissue under normal physiological conditions, but exhibits an endogenous neuroprotective effect in case of brain damage, is involved in signal transduction of G-protein coupled receptor, and regulates apoptosis and cell survival by electron transfer of cytochrome C in mitochondria, as a mechanism of action for the above. It has been reported that Ngb is particularly highly expressed selectively in a neuron of brain and is not only an endogenous neuroprotective factor, but also has a function of promoting nerve regeneration at the same time, thus playing a protective role against Alzheimer's diseases and stroke (Greenberg et al., 2008) (Yu et al., 2013B). Recently, the E2(ER)-inducible Ngb enhancement effect has been reported. Since such Ngb is a pharmacologically regulatable target, enhancement of Ngb signal through drugs is expected to be very effective and useful in brain protection treatment (Jin et al., 2011).

Meanwhile, Prunus cerasoides is a plant of the genus Prunus of the Rosaceae family, and Prunus cerasoides used in the experiment of the present disclosure was obtained from overseas biological material hub center. Prunus cerasoides is also called wild Himalayan cherry or sour cherry. Prunus cerasoides is known to inhabit the Himalayan mountains in Himachal Pradesh province of North India, southwestern of China, Burma province, Thailand, etc. A water-soluble branch extract of the Prunus cerasoides plant has been reported to be used in India to prevent abortion (Kirtkar, K.R et al., 1975). Previous studies have revealed that Prunus cerasoides contains flavonoids, steroids, terpene, etc. Prunus cerasoides contains glucogenkwanin as a flavone family, naringenin as flavanones, and genistein and prunetin as isoflavonoids. Prunus cerasoides contains β-sitosterol and ursoric asid as steroids and terpenes. A study on naringenin, prunetin, β-sitosterol, and ursolic acid are conducted, as a study on components identified from Prunus cerasoides (Kim S et al., 2013) (Tsutsui T et al., 2003) (Touillaud MS et al., 2005) (Gu G et al., 2012). A study of the estrogenic and Ngb upregulation activity of Prunus ceresoides extract was published in December 2016 as a Master's degree dissertation by So-Dam Kim from the Graduate School Sookmyung Women's University (XP055665003).

### [Disclosure]

### [Technical Problem]

The object of the present disclosure is to provide a composition for neuroprotection having an effect of treatment, alleviation and prevention of cerebral infarction, through the activation of neuroglobin (Ngb).

The present disclosure has found that a Prunus cerasoides extract or fraction has a neuroprotective effect through neuroglobin (Ngb) activation, and based on these results, the object of the present disclosure is to provide a composition for neuroprotection, containing a Prunus cerasoides extract or fraction, as active ingredients.

The present disclosure has firstly found that a Prunus cerasoides extract or fraction has a neuroprotective efficacy and has proven that a Prunus cerasoides extract or fraction has a neuroprotective efficacy through the activation of neuroglobin (Ngb).

The present invention, which is defined by the claims, relates to:
- A pharmaceutical composition comprising a *Prunus cerasoides* extract or fraction thereof, as active ingredient for use in neuroprotection for the treatment and prevention of a cerebral infarction through the activation of neuroglobin (Ngb), and to:
- A food composition comprising a *Prunus cerasoides* extract or fraction thereof, as active ingredient for use in neuroprotaction for the improvement and prevention of a cerebral infarction through the activation of neuroglobin (Ngb).

### [Technical Solution]

In order to achieve the above object, the present disclosure provides a pharmaceutical composition for neuroprotection for any one of treatment and prevention of a cerebral infarction, through the activation of neuroglobin (Ngb), containing a Prunus cerasoides extract or fraction, as active ingredients.

Also, the present disclosure provides a food composition for neuroprotection for any one of improvement and prevention of a cerebral infarction, through the activation of neuroglobin (Ngb), containing a Prunus cerasoides extract or fraction, as active ingredients.

A Prunus cerasoides extract in the composition is preferably obtained by ultrasonic extraction at 30°C to 60°C for 2 to 4 days, using methanol or ethanol as an extraction solvent.

In the food composition, the food may have a form of any one of tablets, capsules, powders, granules, liquids, and pills. In addition, the food may have a form of any one of beverages, powdered beverages, solids, chewing gum, tea, vitamin complexes, and food additives.

### [Advantageous Effects]

The present disclosure revealed that a Prunus cerasoides extract and fraction has the neuroprotective effect through the activation of neuroglobin (Ngb). The present disclosure provides a composition for neuroprotection, containing a Prunus cerasoides extract or fraction, as active ingredients, based on these results.

### [Description of Drawings]

FIG. 9 is a result of testing whether a Prunus cerasoides extract changes Ngb gene transcription activity in SKNSH cells transfected with Ngb-luc plasmid, showing an increase in Ngb gene transcription activity by a Purunus cerasoides extract.

FIG. 10 is a result of testing whether a Prunus cerasoides extract changes Ngb gene transcription activity in N2a cells transfected with Ngb-luc plasmid, showing an increase in Ngb gene transcription activity by a Purunus cerasoides extract.

FIG. 11 is a result of confirming a change of Ngb gene expression in N2a cells treated with vehicle (0.1% DMSO), a Purunsu cerasoide extract.

FIG. 12 is a result of confirming the neuroprotective effect of a Purunus cerasoides extract in a hypoxic model in vitro, showing a result of significantly increasing a viability of cell at a concentration of 10⁻⁷ to 10⁻⁶ g/ml under a condition of hypoxic culture when treated with a Prunus cerasoides extract.

FIG. 13 is a result of confirming the neuroprotective effect of a Purunus cerasoides fraction in a hypoxic model in vitro, showing a result of effectively improving viability and toxicity of cell at a concentration of 10⁻⁶ g/ml under a condition of hypoxic culture when treated with of a Prunus cerasoides fraction.

FIG. 14 is a result of testing whether a Prunus cerasoides extract reduce cerebral infarction due to stroke in a mouse stroke model, showing a result of measuring the size of the cerebral infarction after 45 minutes occlusion of the middle cerebral artery and 24 hours of reperfusion.

### [Best Mode]

In the present disclosure, it has been found that a Prunus cerasoides extract and fraction has a neuroprotective effect through the activation of neuroglobin (Ngb). The present disclosure provides a composition for neuroprotection, containing a Prunus cerasoides extract and fraction, as active ingredients, based on these results.

### 2. Composition for neuroprotection containing Prunus cerasoides extract or fraction as active ingredients

### (1) Prunus cerasoides extract or fraction

In the present disclosure, a Prunus cerasoides extract may be preferably obtained by extraction at 10 to 80°C, using water, alcohol, or an organic solvent having 1 to 4 carbon atoms. More preferably, the Prunus cerasoides extract may be obtained by a method comprising: extracting by adding an extraction solvent to Prunus cerasoides; filtering the extract; concentrating the filtrate under reduced pressure; and drying the concentrate if necessary. In addition, fractions may be obtained from the extracts obtained thus. Each step will be described in more detail as follows.

### 1) The step of extracting

Extraction was performed by adding an extraction solvent to the mixture of bark, wood, and leaves of Prunus cerasoides. At this time, as the extraction solvent, water, alcohol, or an organic solvent having 1 to 4 carbon atoms may be used, and water or a lower alcohol having 1 to 3 carbon atoms is preferably used. More preferably, methanol or ethanol is used. It is preferable to perform the extraction with the extraction solvent which is added 1 to 20 times of the amount of the dried Prunus cerasoides, and it is more preferable to perform the extraction by adding 5 to 15 times of the amount of the dried Prunus cerasoides. As extraction methods, methods such as shaking extraction, soxhlet extraction, reflux extraction, ultrasonic extraction using an ultrasonic generator, and an automatic extractor may be used. An extraction temperature is preferably 10 to 80°C, more preferably 20 to 70°C, and most preferably 30 to 60°C. An extraction time is not limited, but in the case of ultrasonic extraction, extraction is preferably performed at 50°C for 2 to 4 days, and in the case of using an automatic extractor, extraction is performed for 20±5 minutes. As the automatic extractor, for example, an ASE300 accelerated extractor (DIONEX Corporation), etc., may be used.

In a preferred embodiment of the present disclosure, ultrasonic extraction is performed at 50°C for 2 to 4 days using 95% or more of methanol. In another preferred embodiment, when using an ASE300 accelerated extractor (DIONEX Corporation), extraction is performed at 50°C and 1500 psi for 20 minutes using N2 gas.

### 2) The step of filtering

The extract obtained in the above step is filtered using a porous filtering material. Filtration is to filter out solids, foreign matter, etc., and any filtering material suitable for this purpose may be used without limitation. For example, filter paper, glass filter, glass fiber, cotton, cotton wool, nylon, porous metal, metal fiber, and the like may be used.

### 3) The step of concentrating under reduced pressure

If left for a long time after filtration, a precipitate may be formed, so it is desirable to concentrate immediately after filtration. The concentration under reduced pressure is preferably performed by using a vacuum concentrator under reduced pressure or a rotary evaporator, etc., but is not limited thereto. After being completely concentrated under reduced pressure, the concentrate may be put into a vial and sealed under reduced pressure, or the concentrate may be sealed and stored after further drying steps below.

### 4) The step of drying

After concentration, a drying step may be further added as needed. The drying method is not particularly limited, but preferably, a method such as drying under reduced pressure, vacuum drying, boiling drying, spray drying, freeze drying, etc., may be used.

### 5) The step of obtaining fractions

Fractions were obtained by obtaining extracts for each stem and leaf in the same manner as described above, and then applying the obtained extract to an open column eluted with a methanol solvent. For the stem extract and leaf extract, 16 fractions and 15 fractions were obtained, respectively.

### (2) Validation

In the present disclosure, a gene reporter evaluation test (Neuroglobin-responsive gene transcription test), an evaluation of the effect on the transcriptional activity of an intrinsic hormone-reactive target gene in a cell, and evaluation of neuroprotecive action through cytotoxicity and viability in hypoxia induction (Oxygen glucose deprivation, OGD), and an evaluation of effect in the in vivo ischemic stroke model through middle cerebral artery occlusion (MCAO) surgery, an evaluation of neuroprotective effect through change in Ngb protein expression level in an animal model undergoing ovariectomy (OVX), an evaluation of the effect on blood metabolic markers and liver levels in an animal model undergoing ovariectomy (OVX), and an evaluation of the effect on behavioral neuroprotection in an animal model that received the extract were performed.

Through these experiments, the present disclosure found that a Prunus cerasoides extract or fraction has the activation of neuroglobin (Ngb), a neuroprotective effect, a neuroprotective effect through reduction in the size of cerebral infarction, a neuroprotective effect through an increase in the expression level of Ngb, an effect of improving neutral fat level and liver level, and an effect of improving depression, anxiety, and neurobehavioral symptoms on motility. It was firstly found and proved in the present disclosure that a Prunus cerasoides extract or fraction exhibits a neuroprotective efficacy or neuroglobin (Ngb) activation.

In addition, in the present disclosure, the safety was confirmed through an acute toxicity test of a Prunus cerasoides extract.

Based on the experimental results, a composition for neuroprotection according to the present disclosure may be used as a pharmaceutical composition or a food composition for treatment, alleviation and prevention of a stroke, a cerebral infarction, thrombosis, cerebral degenerative disease, vascular dementia, memory loss, short-term memory impairment, a concentration disorder, anxiety, nervousness and the like, and for brain damage protection, brain function improvement, and cranial nerve cell protection, through the activation of neuroglobin (Ngb).

In addition, recently, a signal association between Ngb and female hormone receptor (ER) in nerve cells has been reported. After the action of estrogen-inducible neuroglobin was first reported in neurons by a De Marinis group (De Marinis, Ascenzi et al. 2010), an increase in the expression of Ngb by estrogen-estrogen receptor (ER)α binding in neurons (SKNBE, NT2) was reported (Guglielmotto, Reineri et al. 2016), and an increase in Ngb induced by the p38 MAPK signaling mediated via ERβ was found (De Marinis, Marino et al. 2011).

In addition, mitochondrial respiration promoting action as a mechanism of action of ERβ has been recently reported (Ponnusamy, Tran et al. 2016), and it is predicted that the possibility of interaction between Ngb and ERβ in mitochondria is very high. According to bioinformatics information, Ngb is estimated to be highly expressed in specific areas of the brain (locus coeruleus, occipital cortex, lobe amygdala), carotid artery, and cardiac muscle cells (Guglielmotto, Reineri et al. 2016), and it can be confirmed that Ngb is very direct treatment target for brain and cardiovascular disease research in women, and it is predicted that Ngb may be a very effective and sensitive target, especially in brain and cardiovascular disease research in menopausal women. Therefore, the composition for neuroprotection according to the present disclosure may be particularly suitably used to protect the cranial nerves of women, particularly menopausal women, and may also be used to relieve menopausal symptoms related to the cranial nerves.

### (3) Pharmaceutical composition

The pharmaceutical composition for neuroprotection according to the present disclosure comprises a Prunus cerasoides extract or fraction as active ingredients.

The pharmaceutical composition according to the present disclosure may further contain suitable carriers, excipients, and diluents conventionally used in the preparation of the pharmaceutical composition in addition to the above active ingredient. The pharmaceutical composition according to the present disclosure may further contain other pharmaceutically active ingredients or active mixture.

The pharmaceutical composition according to the present disclosure may be formulated and used in the form of oral dosage such as powders, granules, tablets, capsules, suspensions, emulsions, syrups, aerosols, etc., external preparations, suppositories, and sterile injectable solutions, respectively, according to conventional methods. Carriers, excipients and diluents that may be included in the composition include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oils. In the case of formulation, it is formulated using diluents or excipients such as fillers, extenders, binders, wetting agents, disintegrants, and surfactants that are usually used. Solid formulation for oral administration includes tablets, pills, powders, granules, capsules, etc., and these solid formulations are prepared by mixing at least one excipient, for example, starch, calcium carbonate, sucrose or lactose, gelatin, etc., with the composition. In addition to simple excipients, lubricants such as magnesium stearate and talc are also used. A liquid formulation for oral administration may be suspensions, oral liquids, emulsions, syrups, etc., and include various excipients, for example, a wetting agent, a sweetener, an aromatic, a preservative, etc., in addition to water and liquid paraffin which are a simple diluent commonly used. Formulation for parenteral administration includes sterilized aqueous solutions, non-aqueous solutions, suspensions, emulsions, lyophilized formulations, and suppositories. As the non-aqueous solvent and suspending agent, propylene glycol, polyethylene glycol, vegetable oil such as olive oil, and injectable ester such as ethyl oleate may be used. As a base for suppositories, Witepsol, macrogol, Tween 61, cacao butter, laurin, glycerogelatin, etc., may be used.

A preferred dosage of the pharmaceutical composition according to the present disclosure is varied with state and weight of a patient, severity of diseases, drug forms, an administration route, and duration, but may be properly selected by a person skilled in the art. However, for a desirable effect, it is recommended to administer a Prunus cerasoides extract at 0.0001 to 1000 mg/kg per day. The administration may be carried out once a day, or may be divided several times. The above dosage does not in any way limit the scope of the present disclosure.

The pharmaceutical composition according to the present disclosure may be administered to mammals such as rats, mice, livestock, and humans by various routes. All modes of administration may be expected and may be administered, for example, by oral, rectal or intravenous, intramuscular, subcutaneous, intrauterine dura mater or Intracerebroventricular injection.

The definitions of the terms for the excipients, binders, disintegrants, lubricants, mating agents, flavoring agents, etc., of the present disclosure are described in documents known in the art, and include those having the same or similar functions.

### (4) Food composition

The pharmaceutical composition for neuroprotection according to the present disclosure comprises a Prunus cerasoides extract or fraction, as active ingredients. The food composition comprises 0.0001 to 20% by weight of the active ingredients based on the total weight.

These foods particularly include health functional foods. "Health functional food" as defined in the present disclosure refers to a food prepared and processed by using raw materials or ingredients having useful functions for the human body, and the term "functional" refers to ingestion for the purpose of controlling nutrients or physiological functions for the structure and function of the human body or obtaining useful effects for health purposes such as medical effects. The health functional food may have a form of any one of tablets, capsules, powders, granules, liquids, and pills.

In addition, the food composition according to the present disclosure may be a food composition in which functional ingredients are added to various foods or beverages. The food may have a form of any one of beverages, powdered beverages, solids, chewing gum, tea, vitamin complexes, and food additives.

The food composition according to the present disclosure is not particularly limited to other ingredients other than those containing the active ingredient as an essential ingredient, and may additionally contain various ingredients of various flavoring agents or natural carbohydrates, etc., such as ordinary foods and beverages. Examples of the aforementioned natural carbohydrates are monosaccharides, for example, glucose, fructose, etc.; disaccharides, for example, maltose, sucrose, etc.; and polysaccharides, for example, common sugars such as dextrin and cyclodextrin, etc., and sugar alcohols such as xylitol, sorbitol, and erythritol. As flavoring agents other than those described above, natural flavoring agents (taumatin, stevia extract (e.g., rebaudioside A, glycyrrhizin, etc.)) and synthetic flavoring agents (saccharin, aspartame, etc.) may be advantageously used. The proportion of the natural carbohydrate is generally about 1 to 20g, preferably about 5 to 12 g per 100 mL of the composition according to the present disclosure.

In addition to those mentioned above, the food composition according to the present disclosure may comprises various nutrients, vitamins, minerals (electrolytes), flavoring agents such as synthetic flavors and natural flavoring agents, coloring agents and heavy weight agents (cheese, chocolate, etc.), pectic acid and a salt thereof, alginic acid and a salt thereof, organic acids, protective colloidal thickeners, pH adjusters, stabilizers, preservatives, glycerin, alcohols, carbonates used in carbonated beverages, etc. In addition, the food composition according to the present disclosure may comprise flesh for the production of natural fruit juice and fruit juice beverage and vegetable beverage. These components may be used independently or in combination. The proportion of these additives is not so important, but is preferable to be generally selected from the range of 0 to about 20% by weight based in a total weight of the food composition according to the present disclosure.

Hereinafter, the present disclosure will be described in more detail through specific experimental examples and working examples. However, these experimental examples and working examples are only for illustrative purposes and the scope of the present disclosure is not limited to these examples.

### [Experimental examples]

### (1) Preparation of Prunus cerasoides extract and fraction

99.9% of methanol was used as the extraction solvent, and an ultrasonic extractor (BRANSON) was used. 200 g of a mixture of bark, wood and leaves of Prunus cerasoides samples, dried well in the shade, was extracted for 3 days at 50°C using 1.5 L of methanol. {(15 minutes for ultrasonic grinding, 2 hours for standing) x 10 times/day} x 3 days.

After extraction, the solid material was filtered through a degreased cotton swab funnel. If left to stand for a long time after filtration, a precipitate was formed, so the filtrate was completely concentrated under reduced pressure at 45°C immediately after formation of the precipitate. The sample concentrated under reduced pressure was sealed in a vial and stored in a low temperature room (-4°C) to use in the experiment.

In addition, the stem and leaf extracts obtained after obtaining the extracts for the stems and leaves in the same manner as described above were applied to an open column eluted with a methanol solvent to obtain 16 fractions and 15 fractions, respectively. These fractions were used in the experiment.

### (2) Experimental method

### 1) Test for evaluation of gene reporter: Neuroglobin (Ngb) responsive gene transcription test

For the Ngb-luciferase assay, a human neuroblastoma cell line (SKNSH) and a mouse neuroblastoma cell line (N2a) transfected with the promoter plasmid and over-express the Ngb gene were used. Upon seeding, cells were cultured in 10% FBS DMEM medium, and seeded in 96-well plates at a concentration of 3 X 10⁴ cells/well. When the cells reached about 45 to 50% confluency, the medium was replaced with 2% FBS DMEM medium. In the case of the Ngb-luciferase assay, drug treatment was performed when cells reach about 80% confluency, and as a negative control group, 0.1% dimethyl sulfoxide (DMSO) and various concentrations of Prunus cerasoides (stock solution dissolved in DMSO) were diluted in a medium, and then were treated with the culture cells. After culturing for 24 hours, the cell culture was stopped, and a water-soluble cell extract was obtained by using passive lysis buffer (Promega, USA). The activity of luciferase present herein was quantitatively measured by using a Luciferase Assay System (Promega; including luciferase substrate and reaction buffer) and a Luminometer (ELISA reader, Promega, Madison, WI, USA). As for the level of gene activity expressed by the test substance, the level of activity indicated by the negative control (0.1% DMSO) was set as 100%, and the level of the relative activity was shown for final comparative evaluation.

### 2) Evaluation of effects of intracellular hormone-reactive target genes on transcriptional activity

Mouse neuroblastoma cells (N2a) were treated with vehicle (0.1% DMSO) and a Prunus cerasoides extract, and then mRNA was extracted by using Trizol. Using an iScript cDNA synthesis kit (Bio-Rad) or a superscript II reverse transcriptase kit (Invitrogen) or ReverTra Ace^{®} qPCR RT Master Mix with gDNA Remover (TOYOBO), a certain amount of mRNA (0.5 to 1 µg) was reverse-transcribed to obtain cDNA. Real-time PCR was performed by using a pair of primers capable of recognizing cDNA and cDNA of the target gene and a PCR SYBR green kit (Qiagen) reagent to quantitatively measure the expression level of the corresponding gene. In order to compensate for the technical error of the reactions in various stages, the expression level of the housekeeping gene GAPDH was simultaneously measured, and a ratio of the expression level of this gene and the expression level of the target gene was used for the final quantitative analysis.

The primers that recognize GAPDH are forward 5'-CTCTCTGCTCCTCCTGTTCGAC and reverse 5'-TGAGCGATGTGGCTCGGCT. The expression level of human Ngb (hNgb) as a neuroprotective target gene was measured. For real-time RT PCR reaction, primers that recognize hNgb are forward 5'-TGGAAGACCTGTCCTTCACTG and reverse 5'-GAGCAGAGACTCACCCACTG. Real-time PCR was performed in about 40 cycles (95°C; 15 seconds, 60°C; 60 seconds). After each gene expression curve was expressed as a logarithm, a threshold cycle (C_{T}) value was mathematically obtained. The value obtained by incorporating this value into 2-^{ΔΔ}C_{T} represents the relative expression level of a specific gene. The value obtained by dividing the expression level of a specific gene by the expression level of the house keeping gene was selected as the final value for the gene expression level, and the treatment values of the vehicle and the test substance were relatively compared.

### 3) Evaluation of neuroprotective action through cytotoxicity and viability in hypoxia induction (oxygen glucose deprivation, OGD)

Mouse neuroblastoma cells (N2a) and primary cortical neuron cells were placed in an anerobic chamber (PlasLabs) depleted of oxygen, and OGD was performed for 8 hours and 3 hours, respectively. In the case of primary cortical neuron cells, a pure layer of neuron was obtained by using a microscope from cortex isolated from fetuses of pregnant mice on the 14th to 16th days, treated with trypsin for 15 minutes, added horse serum, centrifuged, and separated to cortical neuron by using growth medium. The 24-well plate was coated with poly-D-lysine to seed primary neurons, and replaced with neurobasal medium to which 2% of B-27 was added once every 2 to 3 days. The cells were cultured for 6 to 7 days in an incubator containing 95% air/5% CO2 at 37°C, and then treated with drugs. The OGD induction solution comprised NaCl (110 mM), KCl (5 mM), CaCl₂ (1.8 mM), NaHCO₃ (44 mM), MgSO₄ (0.8 mM), and did not comprise glucose and plasma, with keeping pH at 7.4. In the case of primary cortical neuron cells, a day before OGD induction, vehicle (0.1% DMSO), a Prunus cerasoides extract and fraction was treated in neurobasal medium to which 1% B-27 was added. The wells of the cell culture plate were washed once, and cells were treated with neurobasal medium treated with the drug, and after 24 hours, the wells of the plate were washed once and treated with the OGD solution, a mixed gas of 90% nitrogen, 5% hydrogen and 5% carbon dioxide, was passed through for 30 minutes or more to completely remove oxygen from a bubbling solution. In the case of N2a cell, the wells of the cell culture plate were washed once, and immediately after the OGD solution treated with the drug was applied to the cells, a mixed gas of 90% nitrogen, 5% hydrogen and 5% carbon dioxide was passed through 30 minutes or more to completely remove oxygen from the bubbling solution. After a certain period of time, it was treated with neurobasal medium to which 2% B-27 was added in the case of primary cortical neuron cell and DMEM containing glucose and plasma in the case of N2a cell, transferred to an incubator, and reperfused for 24 hours to confirm the change over time using the WST assay kit (Dojindo) and the LDH assay kit (Biovision). After 24 hours of reperfusion, the media was taken from the well of the plate and an WST assay was performed, the cell viability was evaluated through the absorbance measurement value of 450 nm, or the LDH assay was performed, and the cytotoxicity was evaluated through the absorbance measurement value of 490 nm and 600 nm. The cell viability and toxicity represented by the test substance were evaluated by finally comparing the level of their relative activity with the level of the cell viability and toxicity represented by the vehicle (0.1% DMSO) set as 100%.

### 4) Evaluation of neuroprotective effect in living ischemic stroke model through middle cerebral artery occlusion (MCAO) surgery

In order to evaluate the neuroprotective effect in the oxidative damage of brain tissue due to cerebrovascular occlusion and reperfusion, mouse MCAO surgery was performed to construct a stroke model. After exposing external carotid artery (ECA) by incising the median neck of a 10-week-old female CD1 mouse, a nylon suture was inserted through the external carotid artery into the internal carotid artery (ICA) and thereby occluded the middle cerebral artery (MCA). After 45 minutes, it was confirmed through 2,3,5-triphenyltetrazolium chloride (TTC) staining that a transient local ischemia model was produced by removing the nylon suture to restore blood flow and causing ischemia/reperfusion damage.

Experimental animals were divided into a vehicle (0.9% NaCl solution, saline) group and a Prunus cerasoides (250 mg/kg) extract group. Two groups were administered orally drugs at 24 hours intervals for 4 weeks before MCAO surgery was performed, and one day after MCAO surgery, brain tissues of mice were obtained and the size of the cerebral infarct was measured. The change in the size of the cerebral infarction indicated by the test substance was evaluated by measuring the change in the relative cerebral infarction size based on the size of the cerebral infarction indicated by the vehicle (0.9% NaCl solution, saline) group.

### 8) Evaluation of safety effect through single-dose acute toxicity test of extract

In order to increase the clinical applicability of plant extracts, a single-dose acute toxicity test was performed. 10-week-old female CD1 mouse was used and fasted from 24 hours before administration. Does of vehicle (1% CMC in saline), and 250 mg/kg, 500 mg/kg and 1000 mg/kg of extract were administered once orally to measure weight change, behavioral observation, and mortality. The experiment was conducted for a total of 2 weeks, and the administration volume was within 5 µl/g.

### (3) Result

### 1) Analysis of transcriptional activity in SKNSH cell and N2a cell through the Ngb response gene of Prunus cerasoides extract

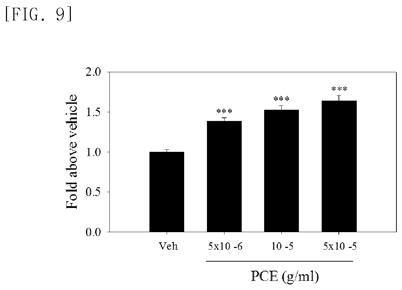

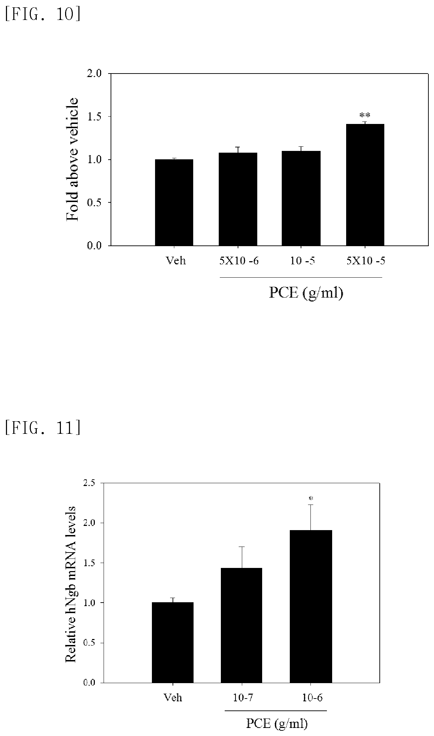

It was tested whether a Prunus cerasoides extract changed the transcriptional activity of Ngb gene in SKNSH and N2a cells transfected with Ngb-luc plasmid, and the results are shown in FIGS. 9 and 10. When SKNSH cells were treated with a Prunus cerasoides extract at a concentration of 5 x 10⁻⁶ g/ml to 5 x 10⁻⁵ g/ml, the transcriptional activity of the Ngb gene was statistically significantly increased from 1.4 to 1.6 times, compared to the vehicle at all concentrations (** p <0.01) (FIG. 9). When N2a cells were treated with the Prunus cerasoides extract at a concentration of 5 x 10⁻⁶ g/ml to 5 x 10⁻⁵ g/ml, the transcriptional activity of the Ngb gene was statistically significantly increased by about 1.4 times at the concentration of 5 x 10⁻⁵ g/ml (*** p <0.001, ** p<0.01) (FIG. 10). From these experimental results, it was found that the Prunus cerasoides extract induces Ngb transcriptional activity at the concentration of 5 x 10⁻⁵ g/ml. In addition, it was confirmed that the extracts exhibit higher Ngb transcription activity than the vehicle (Veh) for two types of cells.

### 2) Induction of Ngb gene expression in N2a cells of Prunus cerasoides extract

It was confirmed Ngb gene expression in N2a cells treated with the vehicle (0.1% DMSO), and a Prunus cerasoides extract. The result is shown in FIG. 11. When N2a cells were treated with the Prunus cerasoides extract at the concentration of 10⁻⁷ g/ml to 10⁻⁶ g/ml, the Ngb gene expression was significantly increased by about 2 times at the concentration of 10⁻⁶ g/ml (FIG. 11). From these experimental results, it was confirmed that the Prunus cerasoides extract induces the expression of Ngb gene.

### 3) Neuroprotective effects of Prunus cerasoides extrat and fraction on hypoxia-inducing cells

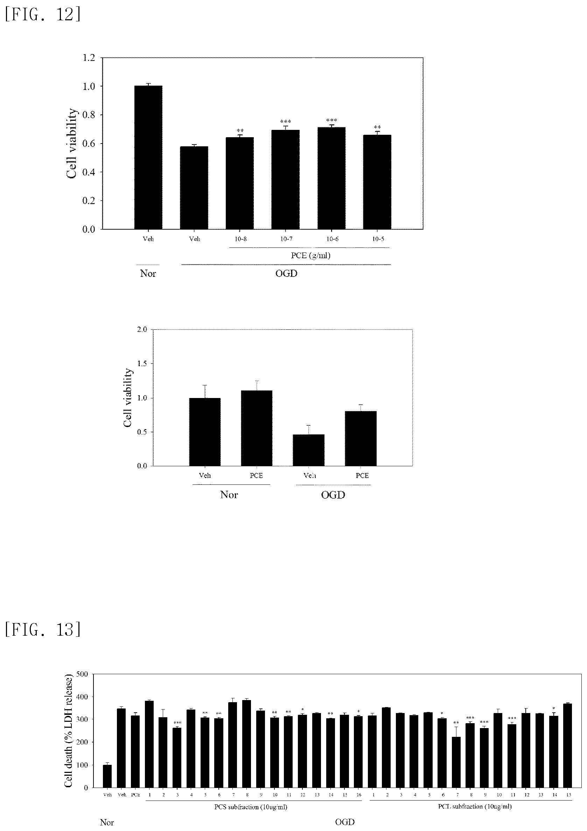

As a result of confirming the neuroprotective effect of a Prunus cerasoides extract in an in vitro hypoxia model, when N2a cells were treated with the Prunus cerasoides extract, the viability of cells at the concentration of 10⁻⁷ to 10⁻⁶ g/ml under hypoxic culture conditions was significantly increased, and when the primary cortical neuron cells were treated with the Prunus cerasoides extract, the viability of cells was increased by about 2 times at the concentration of 10⁻⁶ g/ml under hypoxic culture conditions (FIG. 12).

As a result of confirming the neuroprotective effect of a Prunus cerasoides fraction in the in vitro hypoxia model, when primary cortical neuron cells were treated with the Prunus cerasoides fraction, the fractions of stems 3, 5, 6, 10, 11, 12, 14, and 16, and the fractions of leafs 6, 7, 8, 9, 11, and 14 significantly reduced cytotoxicity by 10 to 30% at the concentration of 10⁻⁵ g/ml under hypoxic culture conditions (FIG. 13). From these experimental results, it could be confirmed that the Prunus cerasoides extract and fraction were effective in increasing cell viability and reducing toxicity under hypoxic culture conditions, and it was evaluated that the neuroprotective effect was excellent (FIGS. 12 and 13).

### 4) Neuroprotective effect by reducing the size of cerebral infarction of Prunus cerasoides

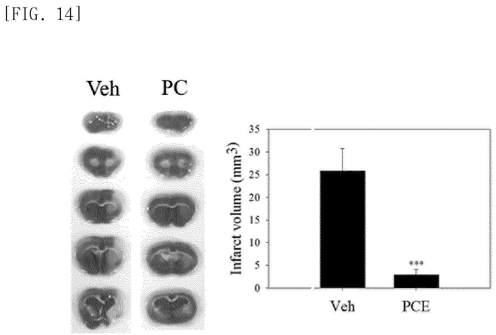

In order to confirm whether a Prunus cerasoides extract exhibited a neuroprotective action, such as reducing cerebral infarction due to stroke, the drug was administered to female CD1 mice by intraperitoneal injection for 4 weeks. After 45 minutes of occlusion of the middle cerebral artery and 24 hours of reperfusion, the size of the cerebral infarction was measured to confirm the neuroprotective effect. The result is shown in FIG. 14. Compared to the vehicle group, a Prunus cerasoides extract group reduced the size of cerebral infarction by about 80% (FIG. 14). From these experimental results, it was found that long-term drug administration of the Prunus cerasoides extract showed a neuroprotective effect by reducing the size of cerebral infarction in a stroke-induced state.

### 8) Effect of single-dose acute toxic safety of Prunus cerasoides extract

A single dose acute toxicity experiment was performed to evaluate the in vivo toxicity of a Prunus cerasoides extract. Female CD1 mice were divided into four groups and administered orally at doses of vehicle (1% CMC saline), and 250 mg/kg, 500 mg/kg and 1000 mg/kg of a Prunus cerasoides extract, and mortality, weight change and behavior were observed for 2 weeks. The result is shown in FIG. 2. Immediately after administration, the movement of the animals decreased, but after 24 hours, the animals showed that normal behavior, and feed and water intake were also observed normally. For a total of 2 weeks of experimentation, the mortality was 0% in all groups including the vehicle group, and the change in body weight between each group was similar. Even at the maximum dose of 1000 mg/kg oral administration, there was no acute toxicity, and so safety was evaluated as high.

**[Table 2]**

| **Group** | **Age** | **Weight (g)** | | **Mortality (%)** |
|---|---|---|---|---|
| | | **Day 0** | **Day 14** | |
| Veh | 12 weeks | 26.06±0.533 | 31.10±0.459 | 0 |
| PC 250 mg/kg | 12 weeks | 25.70±0.868 | 29.68±0.596 | 0 |
| PC 500 mg/kg | 12 weeks | 26.43±0.617 | 31.05±0.865 | 0 |
| PC 1000 mg/kg | 12 weeks | 26.81±0.494 | 31.68±0.331 | 0 |

Evaluation of acute toxicity by a Prunus cerasoides extract.

## Claims

1. A pharmaceutical composition comprising a *Prunus cerasoides extract* or fraction thereof, as active ingredients for use in neuroprotection for the treatment and prevention of a cerebral infarction through the activation of neuroglobin (Ngb).

2. The pharmaceutical composition for use of claim 1, wherein the *Prunus cerasoides* extract is obtained by an ultrasonic extraction at 30°C to 60°C for 2 to 4 days, using methanol or ethanol as an extraction solvent.

3. A food composition comprising a *Prunus cerasoides* extract or fraction thereof, as active ingredients for use in neuroprotection for the improvement and prevention of a cerebral infarction through the activation of neuroglobin (Ngb).

4. The food composition for use of claim 3, wherein the *Prunus cerasoides* extract is obtained by an ultrasonic extraction at 30°C to 60°C for 2 to 4 days, using methanol or ethanol as an extraction solvent.

5. The food composition for use of claim 3 or 4, wherein the food is a health functional food having a form of any one of tablets, capsules, powders, granules, liquids, and pill.

6. The food composition for use of claim 3 or 4, wherein the food has a form of any one of beverages, powdered beverages, solids, chewing gum, tea, vitamin complexes, and food additives.

## Patentansprüche

1. Pharmazeutische Zusammensetzung, umfassend einen *Prunus-cerasoides-Extrakt* oder Fraktion davon als Wirkstoffe zur Verwendung bei Neuroprotektion für die Behandlung und Prävention eines Hirninfarkts durch die Aktivierung von Neuroglobin (Ngb).

2. Pharmazeutische Zusammensetzung zur Verwendung nach Anspruch 1, wobei *der Prunus-cerasoides-Extrakt* durch eine Ultraschallextraktion bei 30 °C bis 60 °C für 2 bis 4 Tage unter Verwendung von Methanol oder Ethanol als Extraktionslösungsmittel erhalten wird.

3. Nahrungsmittelzusammensetzung, umfassend einen *Prunus-cerasoides-Extrakt* oder Fraktion davon als Wirkstoffe zur Verwendung bei Neuroprotektion für die Verbesserung und Prävention eines Hirninfarkts durch die Aktivierung von Neuroglobin (Ngb).

4. Nahrungsmittelzusammensetzung zur Verwendung nach Anspruch 3, wobei der *Prunus-cerasoides-Extrakt* durch eine Ultraschallextraktion bei 30 °C bis 60 °C für 2 bis 4 Tage unter Verwendung von Methanol oder Ethanol als Extraktionslösungsmittel erhalten wird.

5. Nahrungsmittelzusammensetzung zur Verwendung nach Anspruch 3 oder 4, wobei das Nahrungsmittel ein gesundheitsfunktionelles Nahrungsmittel ist, das eine Form von einem beliebigen von Tabletten, Kapseln, Pulvern, Granulaten, Flüssigkeiten und Pille aufweist.

6. Nahrungsmittelzusammensetzung zur Verwendung nach Anspruch 3 oder 4, wobei das Nahrungsmittel eine Form von einem beliebigen von Getränken, pulverförmigen Getränken, Feststoffen, Kaugummi, Tee, Vitaminkomplexen und Nahrungsmittelzusatzstoffen aufweist.

## Revendications

1. Composition pharmaceutique comprenant un extrait de *Prunus cerasoides* ou une fraction de celui-ci, comme ingrédients actifs pour une utilisation en neuroprotection pour le traitement et la prévention d'un infarctus cérébral par l'activation de la neuroglobine (Ngb).

2. Composition pharmaceutique destinée à être utilisée selon la revendication 1, dans laquelle l'extrait de *Prunus cerasoides* est obtenu par une extraction par ultrasons à une température comprise entre 30° C et 60° C pendant 2 à 4 jours, en utilisant du méthanol ou de l'éthanol comme solvant d'extraction.

3. Composition alimentaire comprenant un extrait de *Prunus cerasoides* ou une fraction de celui-ci, comme ingrédients actifs pour une utilisation en neuroprotection pour l'amélioration et la prévention d'un infarctus cérébral par l'activation de la neuroglobine (Ngb).

4. Composition alimentaire destinée à être utilisée selon la revendication 3, dans laquelle l'extrait de *Prunus cerasoides* est obtenu par une extraction par ultrasons à une température comprise entre 30° C et 60 ° C pendant 2 à 4 jours, en utilisant du méthanol ou de l'éthanol comme solvant d'extraction.

5. Composition alimentaire destinée à être utilisée selon la revendication 3 ou 4, dans laquelle l'aliment est un aliment fonctionnel pour la santé ayant une forme de l'un quelconque parmi les comprimés, capsules, poudres, granulés, liquides et pilules.

6. Composition alimentaire destinée à être utilisée selon la revendication 3 ou 4, dans laquelle l'aliment a une forme de l'un quelconque parmi les boissons, boissons en poudre, solides, gomme à mâcher, thé, complexes de vitamines et additifs alimentaires.
